# EUROPEAN PATENT APPLICATION

(11) **EP 4 343 769 A1**
(43) Date of publication of application: **27.03.2024**
(21) Application number: 21940706.1
(22) Date of filing: 18.05.2021
(51) Int. Cl.: G16B 30/10

(54) **INFORMATION PROCESSING PROGRAM, INFORMATION PROCESSING METHOD, AND INFORMATION PROCESSING DEVICE**

(71) Applicant: FUJITSU LIMITED, Kawasaki-shi, Kanagawa 211-8588 (JP)
(72) Inventor: KATAOKA, Masahiro, Kawasaki-shi, Kanagawa 211-8588 (JP); NAGAURA, Ryohei, Kawasaki-shi, Kanagawa 211-8588 (JP); MOGUSHI, Kaoru, Kawasaki-shi, Kanagawa 211-8588 (JP)
(74) Representative: Hoffmann Eitle
(86) International application number: PCT/JP2021/018730
(87) International publication number: WO 2022/244089

(57) **Abstract**

The information processing apparatus calculates a second index indicating a position of an amino acid on a codon file based on a first index indicating positions of a plurality of codons on the codon file with respect to a plurality of codons having different base sequences indicating the same amino acid. Based on the second index, the information processing amino acid sequences that are repeatedly expressed in the codon file based on the second index. The information processing apparatus specifies each codon sequence corresponding to the position of each amino acid sequence repeatedly expressed in the codon file as a codon sequence having homology.

## Description

### FIELD

The present invention relates to an information processing program and the like.

### BACKGROUND

The base sequence of the human genome has been studied, and it has been elucidated that there are 30000 types of proteins constituting the human genome. On the other hand, the types of proteins in microorganisms and the like are considered to be limitless, and a large number of unique codon sequences repeatedly expressed from the target nucleotide sequence have been found. For example, a specific codon sequence that is repeatedly expressed is called a domain, a motif, or the like, and it is important to investigate such a specific codon sequence.

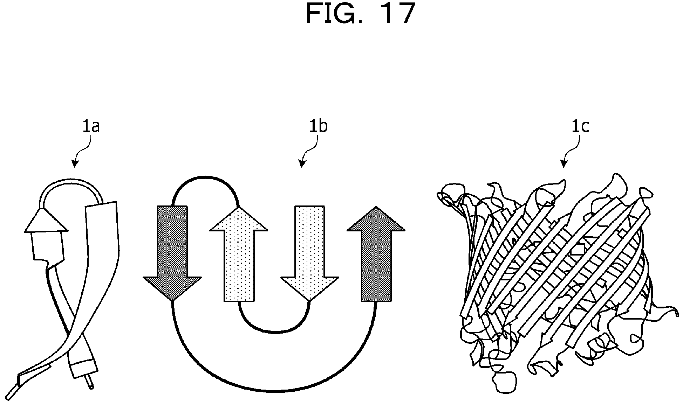

A domain is a part of the sequence or structure of a protein that evolves independently of other parts and has a function. A motif is characterized by a symmetrical sequence of codons. FIG. 17 is a diagram illustrating an example of a motif. As shown in FIG. 17, motifs include β hairpin 1a, Greek key 1b, β barrel 1c (porin or lipocalin), and the like. Folding is the physical process by which a protein chain acquires its native three dimensional structure, usually a biologically functional conformation, in a rapid and reproducible manner.

For example, as a technique for searching for a motif from a base sequence, there is a conventional technique for searching for a motif using a substituted base sequence having a Hamming distance as a key. In addition, there is a conventional technique in which a plurality of sequence cross-sections of an ortholog candidate are extracted from upstream of a transcription start point of a deoxyribonucleic acid (DNA) sequence and a motif candidate is determined.

### [Prior Art Document]

### [Patent Document]

[Patent Document 1] International Publication No. 2005 / 096208
[Patent Document 2] International Publication No. 2020 / 049748
[Patent Document 3] Japanese Patent Application Laid-Open No. 2014 / 112307

### SUMMARY

### [TECHNICAL PROBLEM]

However, in the above-described conventional techniques, there is a problem that a codon sequence that is repeatedly expressed cannot be efficiently searched for.

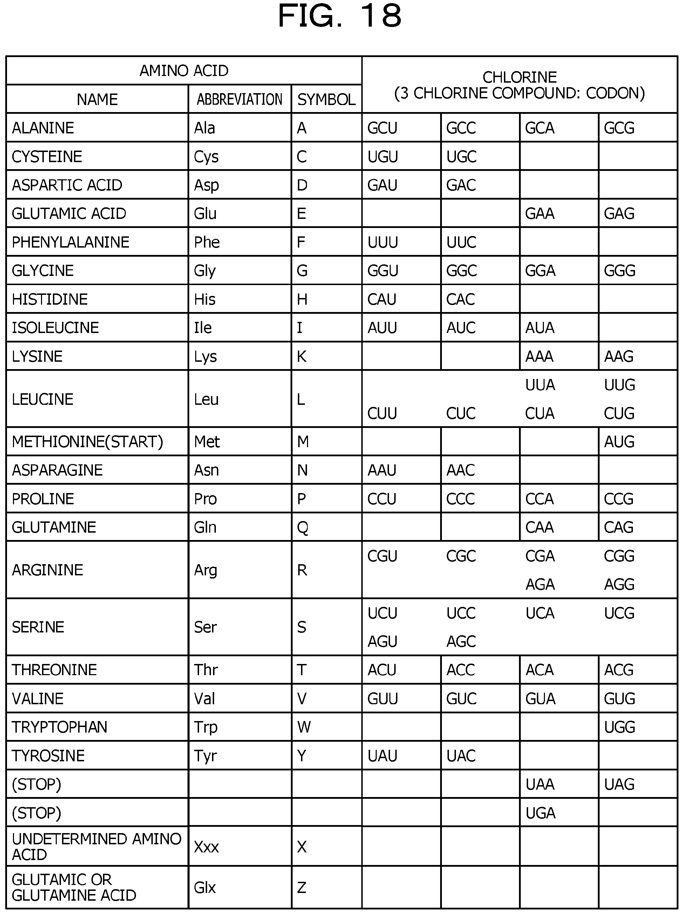

Here, the bases of DNA and RNA (ribonucleic acid) are of four types and are represented by the symbols " A", " G ", " C ", " T " or " U ". Further, 20 kinds of amino acids are determined by a group of three base sequences. The respective amino acids are indicated by the symbols " A " to " Y ". FIG. 18 shows the relationship between amino acids, bases, and codons. A cluster of three base sequences is called a " codon ". A codon is determined by the arrangement of each base, and when the codon is determined, an amino acid is determined.

As shown in FIG. 18, a plurality of types of codons are associated with one amino acid. For example, the amino acid " alanine (Ala) " is associated with the codons " GCU ", " GCC ", " GCA", " GCG ", wherein the codons " GCU ", " GCC ", " GCA ", " GCG " are substantially identical codons. However, the conventional techniques cannot cope with the characteristics of such codons and cannot efficiently search for a codon sequence that is repeatedly expressed.

It is desirable to provide an information processing program, an information processing method, and an information processing apparatus capable of efficiently searching for a codon sequence that is repeatedly expressed.

### [SOLUTION TO PROBLEM]

According to an aspect of the invention, an information processing program that causes at least one computer to execute a process, the process includes calculating a second index indicating a position of an amino acid on a codon file based on a first index indicating positions of a plurality of codons on the codon file with respect to a plurality of codons having different base sequences indicating the same amino acid; identifying positions of amino acid sequences repeatedly expressed in the codon file based on the second index; and specifying each codon sequence corresponding to a position of each amino acid sequence repeatedly expressed in the codon file as a codon sequence having homology.

### [ADVANTAGEOUS EFFECTS OF THE INVENTION]

A codon sequence that is repeatedly expressed can be efficiently searched.

### BRIEF DESCRIPTION OF THE DRAWINGS

FIG. 1 is a diagram (1) for explaining processing of an information processing apparatus according to a first embodiment;
FIG. 2 is a diagram (2) for explaining the processing of the information processing apparatus according to the first embodiment;
FIG. 3 is a functional block diagram illustrating a configuration of the information processing apparatus according to the first embodiment;
FIG. 4 is a diagram illustrating an example of a data structure of a score table;
FIG. 5 is a diagram showing an example of a data structure of a codon file;
FIG. 6 is a diagram illustrating an example of a data structure of a codon permutation index;
FIG. 7 is a diagram illustrating an example of a data structure of an amino acid transposition index;
FIG. 8 is a diagram (1) for explaining processing of a specifying unit;
FIG. 9 is a diagram (2) for explaining a process of a specifying unit;
FIG. 10 is a diagram (3) for explaining the processing of the specifying unit;
FIG. 11 is a diagram (4) for explaining a process of a specifying unit;
FIG. 12 is a diagram illustrating an example of a data structure of search result information;
FIG. 13 is a flowchart illustrating a processing procedure of the information processing apparatus according to the first embodiment;
FIG. 14 is a diagram (1) for explaining a process of the information processing apparatus according to the second embodiment;
FIG. 15 is a diagram (2) for explaining the processing of the information processing apparatus according to the second embodiment;
FIG. 16 is a diagram illustrating an example of a hardware configuration of a computer that realizes the same function as the information processing apparatus according to the embodiment;
FIG. 17 is a diagram showing an example of a motif; and
FIG. 18 is a diagram showing the relationship among amino acids, bases, and codons.

### DETAILED DESCRIPTION OF THE INVENTION

Preferred embodiments of the present invention will be, an information processing method, and an information processing apparatus disclosed in the present application will be described in detail with reference to the drawings. However, the present invention is not limited to these embodiments.

### [Example 1]

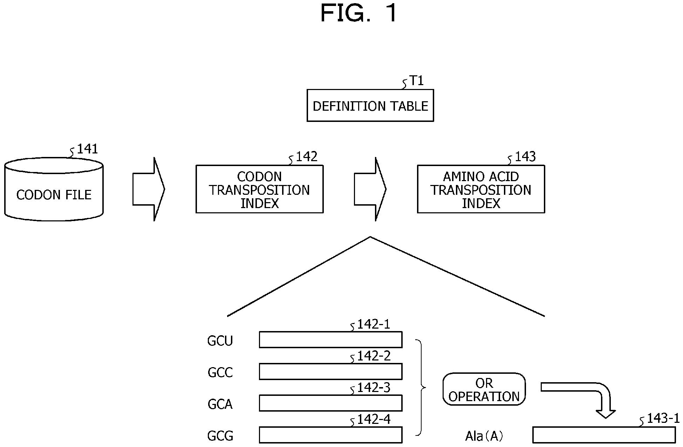

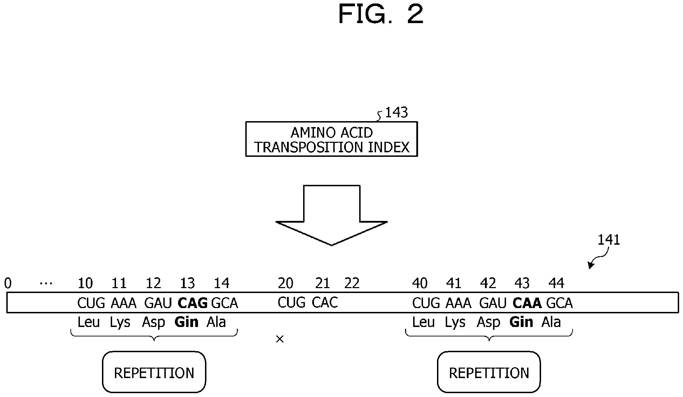

An example of processing performed by the information processing apparatus according to the first embodiment will be described. FIGS. 1 and 2 are diagrams for explaining processing performed by the information processing apparatus according to the first embodiment.

FIG. 1 will be described. The information processing apparatus scans a codon file 141 including information of a base sequence on a codon basis to generate a codon transposition index 142.

The codon permutation index 142 has a bitmap for each type of codon. Since there are 64 types of codons, 64 bitmaps are registered in the codon permutation index 142. Each bit map of the codon permutation index 142 is associated with a type of a codon, an offset, and a flag. At an offset where the flag " 1" of the bitmap is set, it is indicated that a corresponding type of codon is located. In the bitmap, " 0 " is associated with an offset for which a flag is not set.

For example, when the flag " 1" is associated with the offset" n " in the bitmap corresponding to the codon " GCU ", this indicates that the (n + 1) - th codon from the head of the codon file 141 is the codon " GCU ". In the first embodiment, the offset of the first codon of the codon file 141 is set to " 0 ".

The information processor generates an amino acid-inverted index 143 based on the codon-inverted index 142 and the definition table T1. The definition table T1 is a table that defines correspondence between amino acids and codons. As described in FIG. 18, a plurality of types of codons may be associated with the same amino acid.

In the amino acid transposition index 143, a bitmap corresponding to each amino acid is registered. In each bitmap of the amino acid transposition index 143, a type of amino acid, an offset, and a flag are associated with each other. At an offset where a flag " 1" is set in the bitmap, it is indicated that an amino acid of a corresponding type is located. In the bitmap, " 0 " is associated with an offset for which a flag is not set.

A case in which the information processing apparatus generates a bitmap of the amino acid " Ala " among the bitmaps of the amino acids in the amino acid transposition index 143 will be described. The information processing apparatus 100 specifies " GCU ", " GCC "," GCA ", and " GCG " as codons corresponding to the amino acids " Ala " based on the definition table T1.

The information processing apparatus acquires a bitmap 142 - 1 of the codon " GCU ", a bitmap 142 - 2 of the codon " GCC ", a bitmap 142 - 3 of the codon " GCA ", and a bitmap 142 - 4 of the codon " GCG " from the codon permutation index 142. The information processing apparatus performs an OR operation (logical sum) on the bitmaps 142 - 1 to 142 - 4 to generate a bitmap 143 - 1 of the amino acid " Ala ".

That is, when the flag of any one of the offsets " n " of the bitmaps 142 - 1 to 142 - 1 is " 1", the information processing apparatus sets the flag of the offset" n " of the bitmap 143 - 1 to " 1 ". On the other hand, when " 0 " is set to all of the offsets " n " of the bitmaps 142 - 1 to 142 - 1, the information processing apparatus sets " 0 " to the offset" n " of the bitmap 143 - 1. The information processing apparatus repeatedly executes the above processing at each offset.

The information processing apparatus generates bitmaps of other amino acids in the same manner as the bitmap 143 - 1 of the amino acid " Ala ", and registers the bitmap of each amino acid in the amino acid inverted index 143.

FIG. 2 will be described. The information processing apparatus specifies the relationship between the offset of the codon file 141 and the type of amino acid based on the amino acid transposition index 143, and specifies the codon sequences corresponding to the positions of the amino acid sequences that are repeatedly expressed as codon sequences having homology each other.

For example, in the example shown in FIG. 2, the amino acid sequence " Leu, Lys, Asp, Gln, Ala " is repeatedly expressed at offsets 10 to 1440 to 44 and the like of the codon file 141. In this case, the information processing apparatus specifies the codon sequence " CUG, AAA, GAU, CAG, GCA " included in offsets 10 to 14 and the codon sequence " CUG, AAA, GAU, CAA, GCA " included in offsets 40 to 44 as codon sequences having homology.

When the codon sequence " CUG, AAA, GAU, CAG, GCA " is compared with the codon sequence " CUG, AAA, GAU, CAA, GCA ", " CAG " is different from " CAA " in the granularity of the codon. However, since " CAG " and " CAA" correspond to the same amino acid " Gln ", it can be said that the codon sequence " CUG, AAA, GAU, CAG, GCA " and the codon sequence " CUG, AAA, GAU, CAA, GCA " are homologous codon sequences.

As described above, according to the information processing apparatus of the first embodiment, the amino acid inverted index 143 is generated by generating a bitmap of units of amino acids from a bitmap of codons having different base sequences indicating the same amino acid. The information processing apparatus uses the generated amino acid inverted index 143 to specify the relationship with the types of amino acids in the codon file 141, and specifies the codon sequences corresponding to the positions of the amino acid sequences that are repeatedly expressed as codon sequences having homology. This makes it possible to efficiently search for codon sequences that are repeatedly expressed.

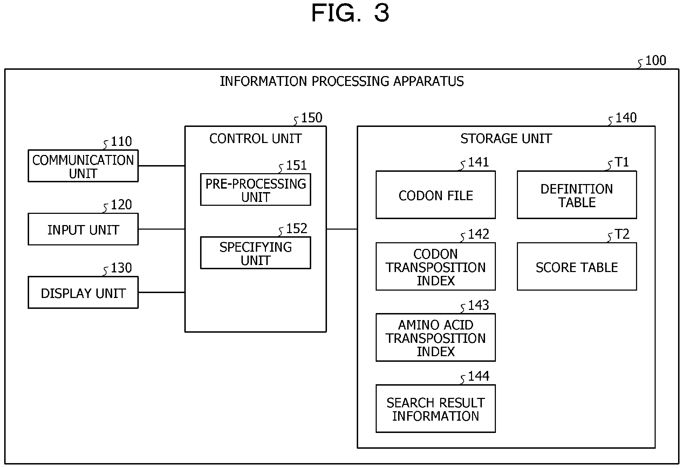

Next, an example of a configuration of the information processing apparatus according to the first embodiment will be described. FIG. 3 is a functional block diagram illustrating the configuration of the information processing apparatus according to the first embodiment. As illustrated in FIG. 3, the information processing apparatus 100 includes a communication unit 110, an input unit 120, a display unit 130, a storage unit 140, and a control unit 150.

The communication unit 110 is connected to an external device or the like in a wired or wireless manner, and transmits and receives information to and from the external device or the like. For example, the communication unit 110 is realized by a network interface card (NIC) or the like. The communication unit 110 may be connected to a network (not illustrated).

The input unit 120 is an input device that inputs various types of information to the information processing apparatus 100. The input unit 120 corresponds to a keyboard, a mouse, a touch panel, or the like.

The display unit 130 is a display device that displays information output from the control unit 150. The display unit 130 corresponds to a liquid crystal display, an organic electro luminescence (EL) display, a touch panel, or the like.

The storage unit 140 includes a definition table T1, a score table T2, a codon file 141, a codon-inverted index 142, an amino-acid-inverted index 143, and search result information 144. The storage unit 140 is realized by, for example, a semiconductor memory element such as a random access memory (RAM) or a flash memory, or a storage device such as a hard disk or an optical disk.

The definition table T1 is a table that defines correspondence between amino acids and codons. The relationship between the amino acids and the codons defined in the definition table T1 is the same as the relationship between the amino acids, the bases and the codons described in FIG. 18.

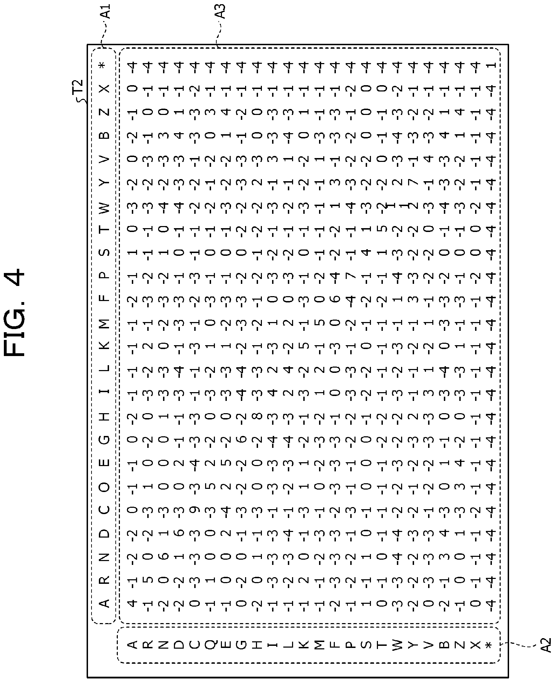

The score table T2 is a table that defines the degree of similarity between amino acids. FIG. 4 is a diagram illustrating an example of a data structure of a score table. The symbols shown in the regions T2 and A1 of the score table A2 shown in FIG. 4 are symbols uniquely indicating the amino acids described in FIG. 18. The numerical value of the region A3 is a score indicating the probability of amino acid replacement, and a higher score indicates a higher degree of similarity.

For example, according to the score table T2 of FIG. 4, the score of alanine " A (Ala) " and threonine " T (Thr) " is " - 4 ". Further, the score of alanine " A (Ala) " and tryptophan " W (Trp) " is " 1 ". Therefore, a pair of alanine and tryptophan shows a higher degree of similarity than a pair of alanine and threonine.

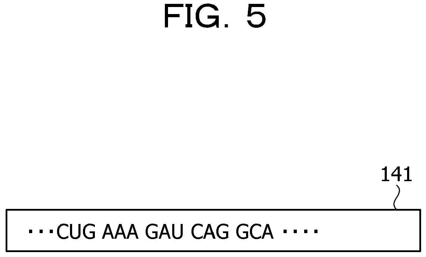

The codon file 141 has information on a base sequence in which a plurality of bases are arranged. FIG. 5 is a diagram illustrating an example of a data structure of a codon file. As illustrated in FIG. 5, the codon file 141 is information in which symbols of a plurality of bases are arranged. A set of three consecutive bases corresponds to one codon.

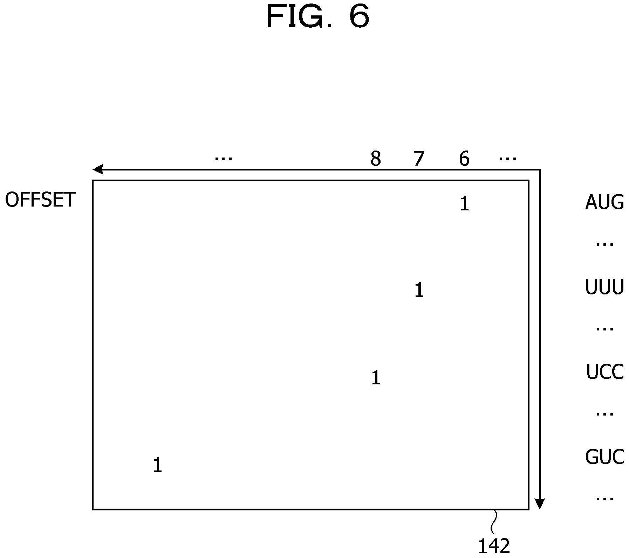

The codon transposition index 142 is information that associates an offset from the head of the codon file 141 with a type of a codon. FIG. 6 is a diagram illustrating an example of a data structure of a codon permutation index. The horizontal axis of the codon permutation index 142 is an axis corresponding to the offset. The vertical axis of the codon permutation index 142 is an axis corresponding to the type of codon.

For example, the offset of the first codon of the codon file 141 is set to " 0 ". When the codon " AUG " is included at the seventh position from the head of the codon file 141, the bit at the position where the column of the offset" 6 " of the codon permutation index 142 and the row of the codon " AUG " intersect is " 1".

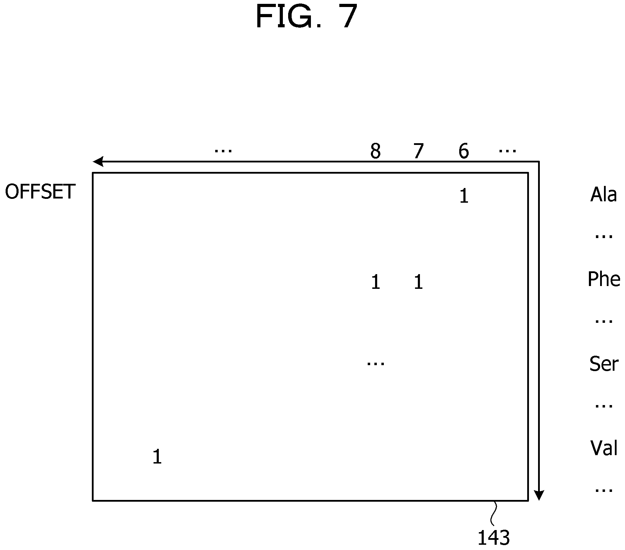

The amino acid transposition index 143 is information that associates an offset from the head of the codon file 141 with the type of amino acid. FIG. 7 is a diagram illustrating an example of a data structure of an amino acid transposition index. The horizontal axis of the amino acid transposition index 143 is an axis corresponding to an offset. The vertical axis of the amino acid transposition index 143 is an axis corresponding to the type of amino acid.

For example, the offset of the first codon (a codon corresponding to any amino acid) of the codon file 141 is set to " 0 ". When any of the codons " GCU ", " GCC ", " GCA ", and " GCG " corresponding to the amino acid " Ala " is included at the seventh position from the head of the codon file 141, the bit at the position where the column of the offset" 6 " of the amino acid transposition index 143 and the row of the amino acid " Ala " intersect is " 1".

The search result information 144 has information on an amino acid sequence (codon sequence) repeatedly expressed in the codon file 141. For example, the search result information 144 holds information on a repeatedly expressed amino acid sequence and a position of the amino acid sequence in association with each other.

The description returns to FIG. 3. The control unit 150 includes a preprocessing unit 151 and a specifying unit 152. The control unit 150 is realized by, for example, a central processing unit (CPU) or a micro processing unit (MPU). Further, the control unit 150 may be implemented by an integrated circuit such as an application specific integrated circuit (ASIC) or a field programmable gate array (FPGA).

The pre-processing unit 151 generates a codon-transposed index 142 and an amino-acid-transposed index 143 based on the codon file 141 and the definition table T1.

An example of a process in which the pre-processing unit 151 generates the codon permutation index 142 will be described. The pre-processing unit 151 selects the type of target codon from the types of codons included in the definition table T1. The pre-processing unit 151 repeatedly executes a process of scanning the codon file 141 from the head thereof at the granularity of the codon (the granularity of a group of three base sequences) and setting the flag " 1" to the offset at which the type of the selected codon appears, thereby generating a bitmap corresponding to the type of the selected codon.

The preprocessing unit 151 generates a bitmap for each of the other codon types in the same manner. The pre-processing unit 151 generates the codon permutation index 142 by setting the bitmap corresponding to the type of each codon in the codon permutation index 142.

Next, an example of a process in which the pre-processing unit 151 generates the amino acid inverted index 143 will be described. The pre-processing unit 151 specifies the type of codon corresponding to the same amino acid and acquires a bitmap corresponding to the specified type of codon from the codon permutation index 142. The pre-processing unit 151 generates a bitmap of an amino acid by performing an OR operation on the acquired bitmap of each codon type.

For example, a case where the pre-processing unit 151 generates a bitmap of the amino acid " Ala " among the bitmaps of the amino acids of the amino acid inverted index 143 will be described. As described with reference to FIG. 1, the preprocessing unit 151 specifies " GCU ", " GCC ", " GCA ", and " GCG " as the codons corresponding to the amino acids " Ala " based on the definition table T1.

The pre-processing unit 151 acquires a bitmap 142 - 1 of the codon " GCU ", a bitmap 142 - 2 of the codon " GCC ", a bitmap 142 - 3 of the codon " GCA ", and a bitmap 142 - 4 of the codon " GCG " from the codon permutation index 142. The preprocessing unit 151 performs an OR operation (logical sum) on the bitmaps 142 - 1 to 142 - 4 to generate a bitmap 143 - 1 of the amino acid " Ala ".

The preprocessing unit 151 generates bitmaps of other amino acids in the same manner as the bitmap 143 - 1 of the amino acid " Ala ", and sets the bitmap of each amino acid in the amino acid inverted index 143 to generate the amino acid inverted index 143.

Next, processing performed by the specifying unit 152 will be described. The specifying unit 152 specifies each position (offset) of an amino acid sequence repeatedly expressed in the codon file 141 based on the amino acid transposition index 143. The specifying unit 152 specifies each codon sequence corresponding to the position (offset) of the amino acid sequence that is repeatedly expressed in the codon file 141 as a codon sequence having homology.

The specifying unit 152 executes the longest match search of the amino acid sequence based on the amino acid transposition index 143, and specifies the longest matching amino acid sequence. When the number of occurrences of the longest matching amino acid sequence is equal to or greater than a preset number of occurrences, the specifying unit 152 searches for the amino acid sequence as an " amino acid sequence candidate ".

For example, as described in FIG. 2, the amino acid sequence " Leu, Lys, Asp, Gln, Ala " is repeatedly expressed at offsets 10 to 14 and 40 to 44 or the like in the codon file 141, and the number of times of expression is equal to or greater than a predetermined number of times of expression. In this case, the specifying unit 152 specifies the codon sequence " CUG, AAA, GAU, CAG, GCA " included in the offsets 10 to 14 and the codon sequence " CUG, AAA, GAU, CAA, GCA " included in the offsets 40 to 44 as codon sequences having homology. The specifying unit 152 registers information on the codon sequence having the specified homology in the search result information 144.

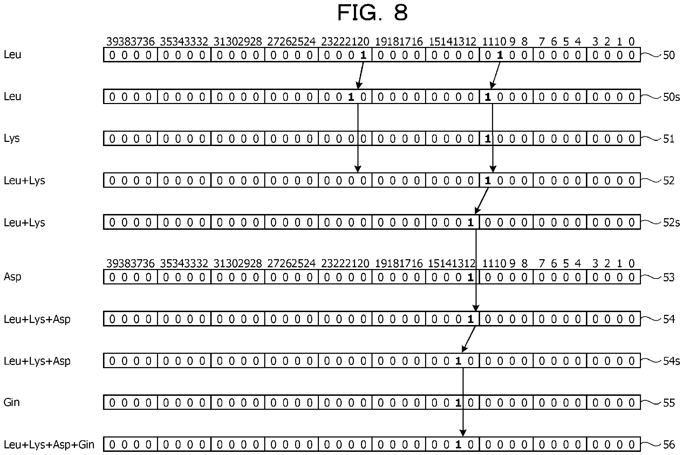

Here, an example of a process in which the specifying unit 152 specifies a continuous amino acid sequence based on the amino acid inverted index 143 will be described. FIG. 8 is a diagram (1) for explaining processing of a specifying unit; In FIG. 8, as an example, a case of specifying whether or not the amino acid sequence " Leu, Lys, Asp, Gln " is included in the codon file 141 will be described.

The specifying unit 152 acquires the bitmap 50 of the amino acid " Leu " from the amino acid inverted index 143. In the bit map 50, the flag " 1" is set to the offsets " 10 " and " 20 ". The specifying unit 152 generates the bitmap 50s by executing the left shift of the bitmap 50. In the bitmap 50s, the flag " 1" is set to the offsets" 11" and " 21".

The specifying unit 152 acquires the bitmap 51 of the amino acid " Lys " from the amino acid inverted index 143. In the bitmap 51, the flag " 1" is set to the offset" 11 ". The specifying unit 152 generates the bitmap 52 by performing an AND operation between the bitmap 50s and the bitmap 51.

In the example illustrated in FIG. 8, since the flag " 1" is set to the offset" 11" of the bitmap 52, it is specified that " Leu, Lys " are continuously present in the offsets " 10 to 11 " of the codon file 141.

The specifying unit 152 generates the bitmap 52s by executing the left shift of the bitmap 52. In the bitmap 52s, the flag " 1" is set to the offset" 12 ".

The specifying unit 152 acquires the bitmap 53 of the amino acid " Asp " from the amino acid inverted index 143. In the bitmap 53, the flag " 1" is set to the offset" 12 ". The specifying unit 152 generates the bitmap 54 by executing an AND operation between the bitmap 52s and the bitmap 53.

In the example illustrated in FIG. 8, since the flag " 1" is set to the offset" 12 " of the bitmap 54, it is specified that " Leu, Lys, Asp " are continuously present in the offsets " 10 to 12 " of the codon file 141.

The specifying unit 152 generates the bitmap 54s by shifting the bitmap 54 to the left. In the bitmap 54s, the flag " 1" is set to the offset" 13 ".

The specifying unit 152 acquires the bitmap 55 of the amino acid " Gln " from the amino acid inverted index 143. In the bitmap 55, the flag " 1" is set to the offset" 13 ". The specifying unit 152 generates the bitmap 56 by performing an AND operation on the bitmap 54s and the bitmap 55.

In the example illustrated in FIG. 8, since the flag " 1" is set to the offset" 13 " of the bitmap 56, it is specified that " Leu, Lys, Asp, Gln " are continuously present in the offsets " 10 to 13 " of the codon file 141.

The specifying unit 152 specifies the longest matching amino acid sequence and specifies the repeatedly expressed amino acid sequence by repeatedly executing the above-described processing for each amino acid sequence. The specifying unit 152 may specify the repeatedly expressed amino acid sequence using another technique.

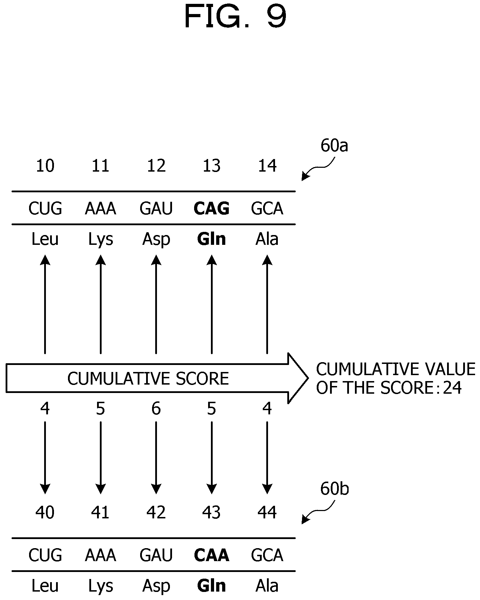

After searching for amino acid sequence candidates by the above-described processing, the specifying unit 152 evaluates the homologies of the amino acid sequence candidates using the score table T2. FIG. 9 is a diagram (2) for explaining the process of the specifying unit; Here, the amino acid sequence candidates 60a and 60b are used for description. The amino acid sequence candidates 60a and 60b are " Leu, Lys, Asp, Gln, Ala ". When " Leu, Lys, Asp, Gln, Ala " is converted into symbols based on the table of FIG. 18 (corresponding to the definition table T1), " L (Leu), K (Lys), D (Asp), Q (Gln), A (Ala) " is obtained.

The specifying unit 152 specifies the score of each of the amino acids based on the score table T2 and accumulates the score to calculate the score of the identity. The score between L (Leu) is " 0 " because it does not exist in the score table T2. The score between K (Lys) is " - 1 " based on the score table T2. The score between D (Asp) is " - 1 " based on the score table T2. The score between Q (Gln) is " 0 " because it does not exist in the score table T2. The score between A (Ala) is " 5 " based on the score table T2. Therefore, the specifying unit 152 calculates the cumulative value " 3 " for the scores of the amino acid sequence candidates 60a and 60b.

When the cumulative value of the score of the amino acid sequence candidate is equal to or greater than a threshold value, the specifying unit 152 specifies the amino acid sequence candidate as an amino acid sequence having a homology relationship. The specifying unit 152 registers the specified result in the search result information 144. The threshold value is preset by an administrator.

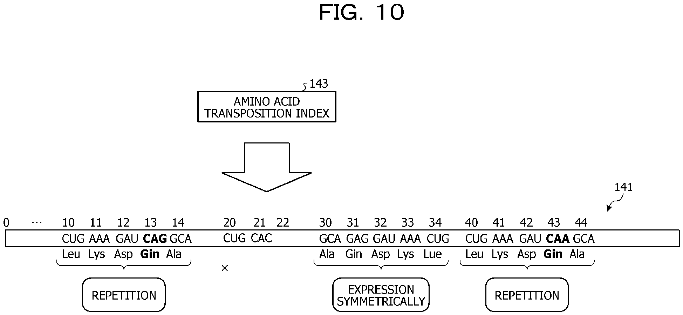

Incidentally, the specifying unit 152 may further specify an amino acid sequence expressed symmetrically with the specified amino acid sequence after specifying an amino acid sequence having a homology relationship. FIG. 10 is a diagram (3) for explaining the process of the specifying unit; For example, the specifying unit 152 specifies " Ala, Gin, Asp, Lys, and Leu " expressed symmetrically to the amino acid sequence " Leu, Lys, Asp, Gln, and Ala " specified in the above-described processing based on the amino acid transposition index 143. In the example illustrated in FIG. 10, the specifying unit 152 specifies the amino acid sequence " Ala, Gin, Asp, Lys, Leu " present at the offset" 30 to 34 " of the codon file 141.

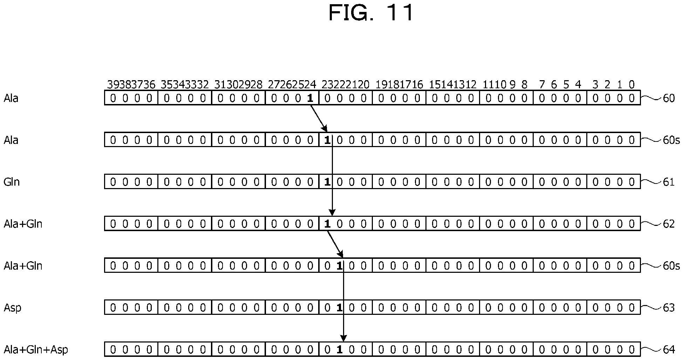

Here, an example of a process in which the specifying unit 152 specifies a symmetric amino acid sequence based on the amino acid inverted index 143 will be described. FIG. 11 is a diagram (4) for explaining the process of the specifying unit; In FIG. 11, as an example, a case of specifying whether or not the symmetrical amino acid sequence " Ala, Gin, Asp (Lys and Leu are omitted) " is included in the codon file 141 will be described.

The specifying unit 152 acquires the bitmap 60 of the amino acid " Ala " from the amino acid inverted index 143. In the bitmap 60, the flag " 1" is set to the offset" 24 ". The specifying unit 152 generates the bitmap 60s by executing the right shift of the bitmap 60. In the bitmap 60s, the flag " 1" is set to the offset" 23 ".

The specifying unit 152 acquires the bitmap 61 of the amino acid " Gln " from the amino acid inverted index 143. In the bitmap 61, the flag " 1" is set to the offset" 23 ". The specifying unit 152 generates the bitmap 62 by executing an AND operation between the bitmap 60s and the bitmap 61.

In the example illustrated in FIG. 11, since the flag " 1" is set to the offset" 23 " of the bitmap 62, it is specified that " Ala, Gln " is continuously present in the offsets " 23 to 24 " of the codon file 141.

The specifying unit 152 generates the bitmap 62s by executing the right shift of the bitmap 62. In the bitmap 62s, the flag " 1" is set to the offset" 22 ".

The specifying unit 152 acquires the bitmap 63 of the amino acid " Asp " from the amino acid inverted index 143. In the bitmap 63, the flag " 1" is set to the offset" 22 ". The specifying unit 152 generates the bitmap 64 by performing an AND operation on the bitmap 62s and the bitmap 63.

In the example illustrated in FIG. 11, since the flag " 1" is set to the offset" 22 " of the bitmap 64, it is specified that " Ala, Gln, Asp " are continuously present in the offsets " 22 to 24 " of the codon file 141.

The specifying unit 152 specifies a symmetrical amino acid sequence by executing the processing described above. The specifying unit 152 registers the specified result in the search result information 144. The specifying unit 152 may output and display the search result information 144 on the display unit 130, or may transmit it to an external device via the communication unit 110.

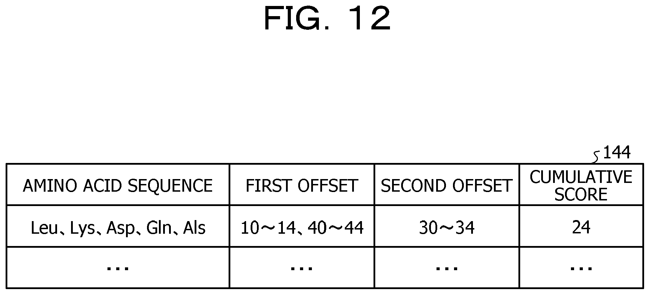

FIG. 12 is a diagram illustrating an example of a data structure of search result information. As illustrated in FIG. 12, the search result information 144 associates an amino acid sequence, a first offset, a second offset, and a cumulative score with one another. The amino acid sequence is a homologous amino acid sequence specified by the specifying unit 152. The first offset indicates an offset of the codon file 141 in which a codon sequence corresponding to a homologous amino acid sequence exists. The second offset indicates an offset of the codon file 141 in which the codon sequence corresponding to the symmetric amino acid sequence exists. The cumulative score is a cumulative value of the score described in FIG. 9.

In FIG. 12, the first offsets corresponding to the amino acid sequences " Leu, Lys, Asp, Gln, Ala " are " 10 to 14 " and " 40 to 44 ". Therefore, the codon sequence corresponding to the offset" 10 to 14 " and the codon sequence corresponding to the offset" 40 to 44 " in the codon file 141 are codon sequences having homology.

In addition, the second offset of the amino acid sequence " Ala, Gin, Asp, Lys, Leu " symmetrical to the amino acid sequence " Leu, Lys, Asp, Gln, Ala " is " 30 to 34 ". Therefore, the codon sequence corresponding to the offset" 30 to 34 " of the codon file 141 becomes a symmetrical codon sequence.

For example, a portion between the homologous amino acid sequence of the search result information and the amino acid sequence symmetrical to this amino acid sequence can be said to be a portion corresponding to a motif. That is, a portion between the first offset" 10 to 14 " and the second offset" 30 to 34 " corresponds to a motif portion.

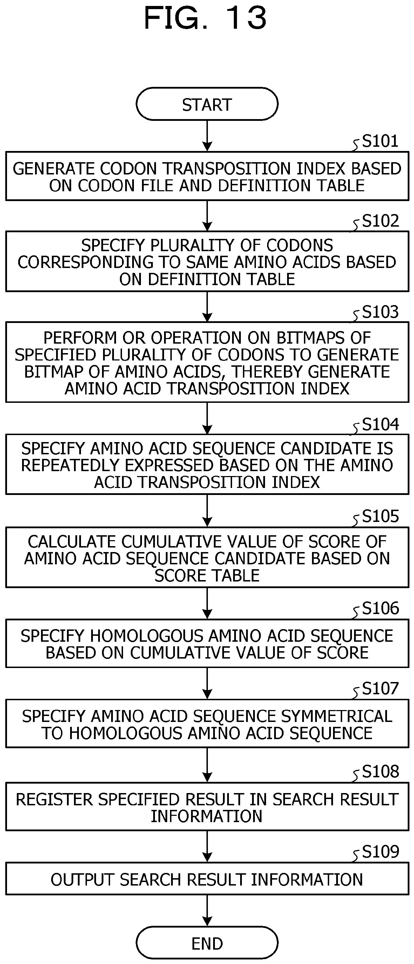

Next, an example of a processing procedure of the information processing apparatus 100 according to the first embodiment will be described. FIG. 13 is a flowchart illustrating a processing procedure of the information processing apparatus according to the first embodiment. As illustrated in FIG. 13, the pre-processing unit 151 of the information processing apparatus 100 generates the codon permutation index 142 based on the codon file 141 and the definition table T1 (step S101).

The preprocessing unit 151 specifies a plurality of codons corresponding to the same amino acids based on the definition table T1 (step S102). The pre-processing unit 151 performs an OR operation on the bitmaps of the specified plurality of codons to generate a bitmap of amino acids, thereby generating the amino acid-inverted index 143 (step S103).

The specifying unit 152 of the information processing apparatus 100 specifies an amino acid sequence candidate that is repeatedly expressed based on the amino acid transposition index 143 (step S104). The specifying unit 152 calculates the cumulative value of the score of the amino acid sequence candidate based on the score table T2 (step S105).

The specifying unit 152 specifies a homologous amino acid sequence (a homologous codon sequence) based on the cumulative value of the score (step S106). The specifying unit 152 specifies an amino acid sequence symmetrical to the homologous amino acid sequence (step S107).

The specifying unit 152 registers the specified result in the search result information 144 (step S108). The specifying unit 152 outputs the search result information 144 (step S109).

Next, effects of the information processing apparatus 100 according to the first embodiment will be described. The information processing apparatus 100 generates the amino acid inverted index 143 by generating a bitmap in units of amino acids from a bitmap of codons having different base sequences indicating the same amino acid. The information processing apparatus 100 specifies the relationship with the types of amino acids in the codon file 141 using the generated amino acid inverted index 143, and specifies the codon sequences corresponding to the positions of the amino acid sequences that are repeatedly expressed as codon sequences having homology. This makes it possible to efficiently search for codon sequences that are repeatedly expressed.

The information processing apparatus 100 evaluates whether or not the amino acid sequences repeatedly expressed in the codon file 141 are homologous amino acids on the basis of a score table T2 defining the degree of similarity between amino acids. Thus, not only the identity of amino acids but also the degree of homology between amino acid sequences can be evaluated.

The information processing apparatus 100 calculates a bitmap of one amino acid corresponding to a plurality of codons by performing a logical sum of the bitmaps of the codon permutation index 142 corresponding to the plurality of codons. Thus, it is possible to easily generate a bitmap of amino acids corresponding to a plurality of codons and generate the amino acid transposition index 143.

### [Example 2]

In Example 1, an amino acid sequence having homology is specified based on the granularity of amino acids, and a codon sequence having homology is specified based on the offset of the specified amino acid sequence, the codon sequence having homology may be specified based on the granularity of codons. In a second embodiment, a process of specifying a homologous codon sequence at the granularity of a codon will be described.

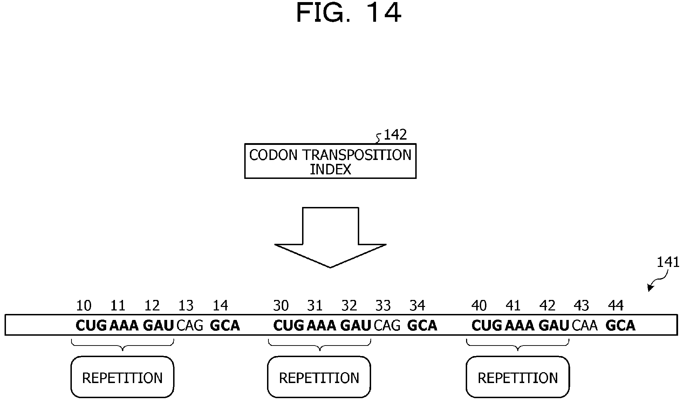

FIG. 14 is a diagram (1) for explaining the processing of the information processing apparatus according to the second embodiment; The information processing apparatus specifies the offset of the codon file 141 and the type of the codon based on the codon transposition index 142, and specifies the codon sequence that is repeatedly expressed. The description of the codon permutation index 142 is the same as the description of the codon permutation index 142 described in the first embodiment.

For example, in the example shown in FIG. 14, the codon sequence " CUG, AAA, GAU " is repeatedly expressed at offsets 10 to 1230 to 32, 40 to 42, and the like of the codon file 141. In this case, the information processing apparatus specifies the codon sequences of offsets 10 to 12,30 to 32 and 40 to 42 as codon sequences having homology. After specifying the codon sequence having homology, the information processing apparatus may specify the amino acid sequence having homology at the granularity of amino acids as described in the first embodiment.

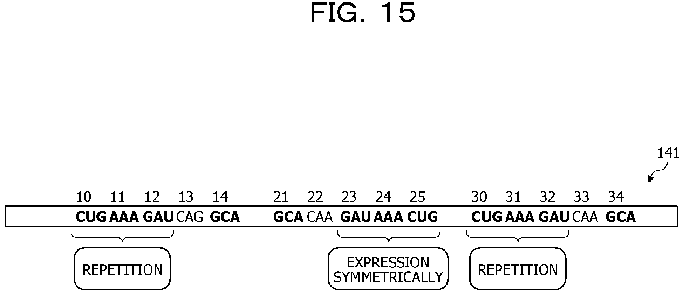

FIG. 15 is a diagram (2) for explaining the processing of the information processing apparatus according to the second embodiment; After specifying a homologous codon sequence, the information processing apparatus may specify a symmetrical codon sequence at the granularity of codons. For example, when the codon sequence having homology is " CUG, AAA, GAU ", the information processing apparatus specifies the symmetrical codon sequence " GAU, AAA, CUG " from the codon file 141. In the example illustrated in FIG. 2, the information processing device specifies that the symmetrical codon sequence " GAU, AAA, CUG " is expressed at offsets 23 to 25.

Note that the processing performed by the information processing apparatus according to the second embodiment to specify the codon sequence such as the longest match using the inverted index is the same as the processing performed using the amino acid inverted index 143 described in the first embodiment, and thus the description thereof is omitted.

The functional block diagram of the information processing apparatus according to the second embodiment corresponds to the functional block diagram of the information processing apparatus 100 illustrated in FIG. 3. The specifying unit 152 illustrated in FIG. 3 additionally executes the processing described with reference to FIGS. 14 and 15.

Although the above-described information processing apparatus 100 specifies a homologous codon sequence and a symmetric codon sequence, and specifies a portion corresponding to a motif or the like, the present invention is not limited thereto, and multiple alignment or the like can be specified. " multiple alignment " refers to alignment or alignment of three or more DNA nucleotide sequences or protein amino acid sequences such that corresponding portions of the sequences are aligned. Usually, it is assumed that the sequences to be aligned have evolutionary relatedness. A molecular phylogenetic tree may be estimated based on based on the results of the multiple based on the results of the multiple alignment.

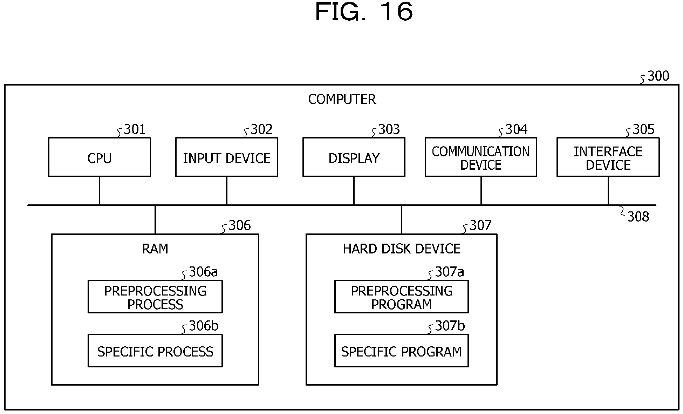

Next, an example of a hardware configuration of a computer that realizes the same function as the information processing apparatus 100 described in the above-described embodiment will be described. FIG. 16 is a diagram illustrating an example of a hardware configuration of a computer that realizes the same function as the information processing apparatus according to the embodiment.

As illustrated in FIG. 16, a computer 300 includes a CPU301 that executes various types of arithmetic processing, an input device 302 that receives data entry from a user, and a display 303. In addition, the computer 300 includes a communication device 304 that exchanges data with an external device or the like via a wired or wireless network, and an interface device 305. The computer 300 also includes a RAM306 for temporarily storing various types of information and a hard disk device 307. The devices 301 to 307 are connected to a bus 308.

The hard disk device 307 includes a preprocessing program 307a and a specific program 307b. CPU301 reads each of the programs 307a to 307d and expands the programs in RAM306.

The preprocessing program 307a functions as a preprocessing process 306a. The specific program 307b functions as a specific process 306b.

The processing of the preprocessing process 306a corresponds to the processing of the preprocessing unit 151. The processing of the specific process 306b corresponds to the processing of the specifying unit 152.

The programs 307a and 307b are not necessarily stored in the hard disk device 307 from the beginning. For example, each program is stored in a " portable physical medium " such as a flexible disk (FD), a CD-ROM, a DVD, a magneto-optical disk, or an IC card that is inserted into the computer 300. Then, the computer 300 may read and execute the programs 307a and 307b.

### [Description of Symbols]

100 Information processing apparatus
110 Communication unit
120 Input unit
130 display unit
140 storage unit
150 control unit

## Claims

1. An information processing program that causes a computer to execute a process, the process comprising:
calculating a second index indicating a position of an amino acid on a codon file based on a first index indicating positions of a plurality of codons on the codon file with respect to a plurality of codons having different base sequences indicating the same amino acid;
identifying positions of amino acid sequences repeatedly expressed in the codon file based on the second index; and
specifying each codon sequence corresponding to a position of each amino acid sequence repeatedly expressed in the codon file as a codon sequence having homology.

2. The information processing program according to claim 1, wherein the process further comprising
evaluating whether an amino acid sequence repeatedly expressed in the codon file is an amino acid having homology based on a table defining a degree of homology between amino acids.

3. The information processing program according to claim 1, wherein the process further comprising:
specifying, from the codon file, a symmetrical amino acid sequence in which an arrangement order of the amino acid sequence is reversed with respect to an amino acid sequence repeatedly expressed in the codon file; and
specifying each codon sequence corresponding to a position of the specified symmetrical amino acid sequence.

4. The information processing program according to claim 1, wherein
the calculating includes calculating a bitmap of the second index of one amino acid corresponding to the plurality of codons by performing a logical sum of the bitmaps of the first index corresponding to the plurality of codons.

5. An information processing method for a computer to execute a process comprising:
calculating a second index indicating a position of an amino acid on a codon file based on a first index indicating positions of a plurality of codons on the codon file with respect to a plurality of codons having different base sequences indicating the same amino acid;
identifying positions of amino acid sequences repeatedly expressed in the codon file based on the second index; and
specifying each codon sequence corresponding to a position of each amino acid sequence repeatedly expressed in the codon file as a codon sequence having homology.

6. The information processing method according to claim 5, wherein the process further comprising evaluating whether an amino acid sequence repeatedly expressed in the codon file is an amino acid having homology based on a table defining a degree of homology between amino acids.

7. The information processing method according to claim 5, wherein the process further comprising:
specifying, from the codon file, a symmetrical amino acid sequence in which an arrangement order of the amino acid sequence is reversed with respect to an amino acid sequence repeatedly expressed in the codon file; and
specifying each codon sequence corresponding to a position of the specified symmetrical amino acid sequence.

8. The information processing method according to claim 5, wherein
the calculating includes calculating a bitmap of the second index of one amino acid corresponding to the plurality of codons by performing a logical sum of the bitmaps of the first index corresponding to the plurality of codons.

9. An information processing apparatus comprising:
a preprocessing unit that calculates a second index indicating a position of an amino acid on a codon file based on a first index indicating positions of a plurality of codons on the codon file with respect to a plurality of codons having different base sequences indicating the same amino acid; and
a specifying unit that:
identifies positions of amino acid sequences repeatedly expressed in the codon file based on the second index, and
specifies each codon sequence corresponding to a position of each amino acid sequence repeatedly expressed in the codon file as a codon sequence having homology.

10. The information processing apparatus according to claim 9, wherein
the specifying unit further executes a process of evaluating whether an amino acid sequence repeatedly expressed in the codon file is an amino acid having homology based on a table defining a degree of homology between amino acids.

11. The information processing apparatus according to claim 9, wherein the specifying unit further causes the computer to execute a process of:
specifying, from the codon file, a symmetrical amino acid sequence in which an arrangement order of the amino acid sequence is reversed with respect to an amino acid sequence repeatedly expressed in the codon file; and
specifying each codon sequence corresponding to a position of the specified symmetrical amino acid sequence.

12. The information processing apparatus according to claim 9, wherein
the preprocessing unit calculates a bitmap of the second index of one amino acid corresponding to the plurality of codons by performing a logical sum of the bitmaps of the first index corresponding to the plurality of codons.
